# EUROPEAN PATENT SPECIFICATION

(11) **EP 0 976 761 B1**
(45) Date of publication and mention of the grant of the patent: **21.05.2003**
(21) Application number: 98309964.9
(22) Date of filing: 04.12.1998
(51) Int. Cl.: C07K 5/06

(54) **Isolation of crystals of alpha-L-aspartyl-L-phenylalanine methyl ester**
Isolierung von Kristallen von Alpha-L-aspartyl-L-phenylalaninmethyl ester
Isolement de cristaux de l'ester méthylique de l'alpha-L-aspartyl-L-phénylalanine

(30) Priority: 29.07.1998 KR 9830674; 30.10.1998 US 183316
(43) Date of publication of application: 02.02.2000
(73) Proprietor: Daesang Corporation, Ichon-si, Kyungki-do (KR)
(72) Inventor: Lee, Man Suk, Kunsan-si, Chonbuk (KR); Choi, Kyung Eun, Sungnam-si, Kyungki-do (KR); Lim, Bun Sam, Seoul (KR)
(74) Representative: Harding, Charles Thomas

(56) References cited:
- EP-A- 0 362 706
- EP-A- 0 476 875
- US-A- 5 728 863

## Description

### Field of the Invention

The present invention relates to producing a sweetening substance, more particularly, to isolation of the α-L-aspartyl-L-phenylalanine methyl ester crystals.

### Background of the Invention

α-L-aspartyl-L-phenylalanine methyl ester, hereinafter referred to as aspartame, is a low-calorie sugar substitute. The aspartame is industrially synthesized in several different processes as well known in the art. The synthesized aspartame is then isolated to provide a commercially applicable form and sometimes further processed to provide various end consumer products, such as granulized aspartame or powder aspartame.

Isolation of the aspartame includes crystallizing the aspartame from aspartame solution, in which the solution turns into slurry of aspartame crystals and solvent. The crystallization is performed by either cooling the aspartame solution or evaporating the solvent. Commonly, a dewatering or separating step separates the solid aspartame crystals from the solvent, and a drying step follows to further remove the solvent from the crystals.

Centrifugation is widely used in separating the solid crystals from the slurry. However, the centrifugation requires a lot of water for washing the solid phase crystals and is not economically desirable. Further, some aspartame crystals may be re-dissolved in the washing water, which may decrease the yield of the aspartame. Alternatively, known in the art is vacuum filtration, in which the solvent drains out by the action of the vacuum and the aspartame crystals remain on the filter bed. This method requires a relatively small amount of water.

It has been known in the art that the removal of water in these dewatering or separating techniques significantly depends on the crystal structures, which in turn is determined by the conditions of the crystallization. Some crystals having needle-like shapes show bad dewaterability and require a longer period of time in the centrifugal or vacuum dewatering. Furthermore, the moisture content within the crystals separated by the dewatering is high, which requires a longer drying process.

There is a suggestion in U.S. Patents Nos. 5,041,607; 5,097,060; 5,543,554 and 5,744,632 that in the cooling type crystallization, removal of the forced flow or stir of the aspartame solution, which is conventionally practiced to homogeneously cool the solution, produces aspartame crystals having good dewaterability. However, cooling crystallization without forced flow or stir takes a longer time than with forced flow and makes temperature gradient within the solution, which may inhibit uniform crystallization of the aspartame. Further, without the forced flow of the solution, it is hard to carry out the crystallization in a continuous mode.

Therefore, there exists a need for a method of separating aspartame crystals having low moisture content, regardless of the crystallization method and the conditions thereof.

### Summary of the Invention

The aforementioned need is solved by the various aspects of the present invention.

The present invention provides a method of isolating α-L-aspartyl-L-phenylalanine methyl ester (aspartame) crystals from a slurry, which comprises a liquid and the aspartame crystals suspended therein. According to the method, the aspartame slurry is prepared by crystallizing the aspartame aqueous solution and introduced into a space enclosed by a filter cloth within a solid chamber at a first pressure. The filter cloth has a large number of permeable perforations through which a part of the liquid of the slurry is preliminarily removed. A resilient tube is provided within the solid chamber and is inflated to squeeze the filter cake at a second pressure and remove the liquid retained in the filter cake through the perforations of the filter cloth. The filter cake is finally discharged from the chamber.

These and other features of the present invention will become more fully apparent from the following description and claims.

### Brief Description of the Drawings

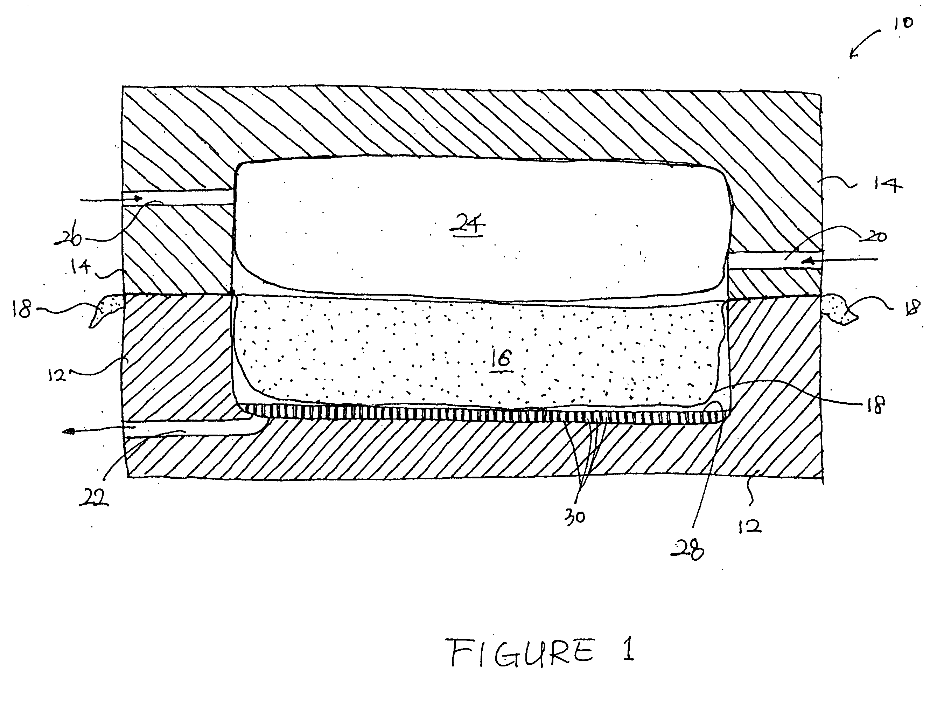

Figure 1 shows a cross sectional view of an exemplary filter in accordance with the present invention. The drawing is intended to illustrate one way of separating the aspartame crystals of the present invention and not to limit the invention.

### Detailed Description of the Preferred Embodiment

Aspartame is synthesized by any known process in the art, and an aspartame aqueous solution is prepared. The aspartame is crystallized from the aspartame aqueous solution with any known crystallization method. The aspartame is crystallized by using either cooling or evaporation.

Before the crystallization, the aspartame aqueous solution is generally heated to adjust the concentration high enough to obtain a decent amount of aspartame crystals in the crystallization. The aspartame solution is heated until aspartame begins to break down and form benzyl-3,6-dioxo-2-piperazine acetic acid or α-L-aspartyl-L-phenylalanine, which is at about 70°C. The initial temperature of the aspartame solution supplied to the crystallization is advantageously from about 30°C to about 70°C. Desirably, the solution is supplied into the inlet at about 40°C to about 60°C.

When using the cooling crystallization, forced flow or stir of the aspartame aqueous solution may or may not be applied. When crystallization process completes, the aspartame solution becomes a slurry, in which the aspartame crystals are suspended within the remaining solution.

The separation of the aspartame crystals from the water in accordance with the present invention is carried out by filtering the slurry while high pressure is applied to the slurry to be filtered. Figure 1 schematically illustrates a filter 10 to be used in connection with the present invention. The filter 10 has a couple of mating vessels 12, 14, which form a chamber 16 inside when they couple together. The lower and upper vessels 12, 14 are advantageously coupled up watertight to avoid any leakage of the slurry or aqueous solution during the pressure filtration of the present invention.

A filter cloth 18 is provided within the chamber 16, advantageously covering the inner surface of the lower vessel 12. The periphery of the filter cloth 18 is advantageously located between the mating areas of the lower and upper vessels 12, 14, which keeps the filter cloth 18 covering the inner surface of the lower vessel 12 to filter the aspartame slurry within the chamber 16, as will be described below. The filter cloth 18 advantageously has the perforations (not shown) less than about 3 m³/m²min. at the pressure of 200Pa.

An inlet passage 20 for introducing the aspartame solution into the chamber 16 is provided. The inlet passage 20 is advantageously formed through the sidewall of the upper vessel 14 to supply the aspartame slurry over the filter cloth 18. An outlet passage 22 for expelling the filtrate, i.e., the liquid in which the aspartame crystals are suspended before the filtration, is provided to the lower vessel 12. The outlet passage 22 is advantageously formed on the bottom of the chamber 16 to facilitate the drainage of the filtrate.

Within the chamber 16, a diaphragm tube 24 to be inflated by compressed water or air is provided and is connected to a passage 26 formed through the upper vessel 14. The diaphragm tube 24 generates pressure when compressed water or air is supplied into the diaphragm tube 24 through the passage 26. The pressure generated by the diaphragm tube 24 presses down and filter the aspartame slurry through the filter cloth 18.

The aspartame slurry is supplied into the chamber 16 of the filter 10 through the inlet passage 20. The aqueous solution in which the crystals are suspended begin to flow through the filter cloth 18 and a filter cake (not shown) is formed in the chamber 16. When the filter chamber 16 is filled up with the filter cake, the supply of the slurry is stopped. An impermeable sealing material (not shown) such as rubber, silicon and teflon plate advantageously seals the mating areas of the lower and upper vessels 12, 14 in order to inhibit the outflow of the slurry.

Alternatively, the aspartame slurry may be pumped into the chamber 16. The pressure of the pumping the slurry is advantageously at least about 0.5 bar and desirably from about 1 bar to about 12 bars. As the pumping pressure increases, the aqueous solution is forced to flow through the filter cloth 18 and drains through the outlet passage 22. When the filter chamber 16 is filled up with the filter cake, the supply of the slurry is stopped.

High pressure is applied onto and squeeze the filter cake by inflating the diaphragm tube 24 as described above. The pressure applied onto the filter cakes is advantageously from about 2 bars to about 20 bars, and desirably from about 4 bars to about 12 bars. Through this pressurized squeeze to the filter cake the aqueous solution retained in the filter cake is filtered through the filter cloth 18. A grid 28 having a large number of small holes 30 may be provided on the bottom of the chamber 16 to enhance the filtration. The filtrate filtered through the filter cloth 18 drains via the outlet passage 22. The thus dewatered filter cake of the aspartame is discharged from the filter chamber and subjected to the following processes including drying.

Before the discharge of the cake, the filter cake is washed with a solvent, which is advantageously supplied through the slurry inlet passage 20 of the filter 10. The filter cake can be washed with the filtrate collected during the pressure filtration in accordance with the present invention. Advantageously, sterilized water washes the filter cakes to reduce the impurities within the cakes. Also, the washing water advantageously spreads evenly over the cake, resulting in uniform cake washing and purity. An additional pressure filtering is advantageously provided to further remove water within filter cake.

Further, advantageously, compressed dry air may be blown to the cake either before or alter the discharge of the cake from the chamber 16. Preferably the compressed air is heated before blowing. When blown before the discharge of the cake, the dry air is advantageously blown through the inlet passage 20 of the filter 10. The compressed dry air blows away the moisture retained within the cake and enables the moisture content minimized. The compressed air is blown with a pressure from about 0.5 bars to about 10 bars for at least about 1 min.

Now the present invention will be further described in terms of the following examples, which are intended to illustrate and not limit the present invention.

### Crystallization of Aspartame

### Example 1

An aspartame aqueous solution for crystallization was prepared, and the initial concentration of aspartame dissolved in the solution was adjusted to 5 wt.% at 60°C. 100 *l* of the prepared aspartame solution was introduced into a crystallizer, which has a crystallizing vessel, a plurality of plates within the vessel and a plurality of jackets around the vessel. 5°C water was supplied to the plates and jackets to cool the aspartame solution contained in the vessel by conductive heat exchange. The aspartame solution was cooled to 25°C without any forced flow or agitation of the solution, thereby aspartame dissolved in the solution crystallized. The crystallized aspartame and the remaining solution were transferred to another vessel, in which they were forcedly stirred by an agitator rotating at 10 rpm to further crystallize the aspartame in the remaining solution as well as to facilitate the separation of solid and liquid phases in the following step while being further cooled to 8°C.

### Example 2

An aspartame aqueous solution for crystallization was prepared, and the initial concentration of aspartame dissolved in the solution was adjusted to 5 wt.% at 60°C. 100 *l* of the prepared aspartame solution was introduced into a crystallizer, which has a crystallizing vessel, a plurality of jackets around the vessel, a rotating agitator provided into the vessel. 5°C water was supplied to the jackets to cool the aspartame solution contained in the vessel by conductive heat exchange. The aspartame solution was cooled to 8°C with forced flow or agitation by the agitator rotating at 30 rpm, thereby aspartame dissolved in the solution crystallized.

### Dewatering Aspartame Crystals

### Example 3

This experiment was conducted by using an "Automatic Pressure Filter", Larox PF 0.1 H2, available from LAROX Oy, Lappeenranta.

100 *l* of aspartame slurry containing 5 wt.% aspartame prepared in Example 1 was pumped into a chamber of the filter at 7 bars for 30 min., which formed aspartame filter cake within the chamber. Compressed water was supplied into a diaphragm tube of the filter. The diaphragm tube inflated and pressed down the filter cake at 5 bars for 2 min. Filtrate of the filtration was collected, and 1 liter of the collected filtrate was supplied into the chamber to wash the filter cake. Compressed water was supplied into the diaphragm tube again, and generated the pressure of 5 bars onto the washed filter cake for 1 min. Sterilized air at the temperature 25°C was blown to the cake at 4 bars for 3 min. The filter cake was finally discharged from the chamber and dried to further remove moisture.

The filter capacity based on the dried solid was 76 kg/m²hr. The moisture content of the discharged cake was 28 wt.%, and the impurities in the cake were 0.21 wt.%.

### Example 4

The same experiment as in Example 3 was repeated except that the sterilized air temperature was 35°C. The filter capacity based on the dried solid was 75 kg/m²hr. The moisture content of the discharged cake was 24.5 wt.%. The impurities in the cake were 0.2 wt.%. 6 min. 40 sec. was taken to dissolve dried aspartame in water at 25°C to make 0.6% aspartame solution.

### Example 5

The same experiment as in Example 3 was repeated except that the aspartame slurry used was prepared according to Example 2. The filter capacity based on the dried solid was 60 kg/m²hr. The moisture content of the discharged cake was 34.3wt.%. The impurities in the cake were 0.34 wt.%. 8 min. 10 sec. was taken to dissolve dried aspartame in water at 25°C to make 0.6% aspartame solution.

### Example 6

The same experiment as in Example 4 was repeated except that the aspartame slurry used was prepared according to Example 2. The filter capacity based on the dried solid was 60.5 kg/m²hr. The moisture content of the discharged cake was 30.1 wt.%. The impurities in the cake were 0.32 wt.%. 8 min. 15 sec. was taken to dissolve dried aspartame in water at 25°C to make 0.6% aspartame solution.

### Example 7

100 *l* of aspartame slurry containing 5 wt.% aspartame prepared in Example 1 was centrifuged at 600G with a centrifugator having the same filter cloth as Example 3. The aspartame crystals centrifuged were spray-washed with 1 liter of drained filtrate which was collected during the centrifugation. The dewatered aspartame crystals by the centrifugation were discharged. The capacity of the aspartame slurry referenced by dried solid crystals was 20.2 kg/m²hr. The moisture content of the discharged cake was 39.2 wt.%. The impurities in the cake were 0.7 wt.%. The time taken in dissolving the aspartame after drying in water to make 0.6% solution at 25°C was 14 min. 30 sec.

### Example 8

The same experiment as in Example 7 was repeated except that the aspartame slurry used was prepared according to Example 2. The capacity of the aspartame slurry referenced by dried solid crystals was 14.3 kg/m²hr. The moisture content of the discharged cake was 48.1 wt.%. The impurities in the cake were 1.03 wt.%. The time taken in dissolving the aspartame after drying in water to make 0.6% solution at 25°C was 18 min. 10 sec.

Table 1 below summarizes dewatering Examples 3 to 8. In Examples 3 to 6, water was removed by compress filtration according to the present invention. In Examples 7 and 8, centrifugation was used in removing the water from the slurry. Examples 5, 6, and 8 used forced agitation during the crystallization, and Examples 3, 4, and 7 do not.

**Table 1**

| | Agitation during Crystallization | Filter Capacity | Moisture Content | Impurity Content | Time for Dissolving at Room Temperature |
|---|---|---|---|---|---|
| Example 3 | No | 76 kg/m²hr | 28 wt.% | 0.21 wt.%. | 7 min. |
| Example 4 | No | 75 kg/m²hr | 24.5 wt.%. | 0.2 wt.%. | 6 min. 40 sec. |
| Example 5 | Yes | 60 kg/m²hr | 34.3wt.% | 0.34 wt.% | 8 min. 10 sec. |
| Example 6 | Yes | 60.5 kg/m²hr | 30.1 wt.% | 0.32 wt.% | 8 min. 15 sec. |
| Example 7 | No | 20.2 kg/m²hr | 39.2 wt.% | 0.7 wt.% | 14 min. 30 sec. |
| Example 8 | Yes | 14.3 kg/m²hr | 48.1 wt.% | 1.03 wt.% | 18 min. 10 sec. |

The filter capacities of crystals obtained by the pressure filtration according to the present invention (Examples 3 to 6) are more than or about three times of those obtained by centrifugation (Examples 7 and 8). The moisture contents of the cakes dewatered by the present invention are all less than those centrifuged. Since the impurities in the slurry are mostly dissolved in the liquid, they are highly dependent on the moisture contents. Accordingly, the impurity contents are also smaller in the crystals obtained by the present invention. The solubility of the aspartame crystals at room temperature was dramatically improved by dewatering in accordance with the present invention.

The properties of the aspartame crystals of Examples 3 to 6 are much more preferable than those of Examples 7 and 8, without reference to the application of forced agitation during the crystallization.

Although the present invention has been described in terms of embodiments, other embodiments will become apparent to those of ordinary skill in the art, in view of the disclosure herein. Accordingly, the present invention is not intended to be limited by the recitation of the embodiments, but is instead intended to be defined solely by reference to the appended claims.

## Claims

1. A method of isolating α-L-aspartyl-L-phenylalanine methyl ester (aspartame) crystals from a slurry comprising a liquid and the aspartame crystals suspended therein, the method comprising:
preparing the aspartame slurry;
introducing the aspartame slurry into an area enclosed by a filter cloth within a solid chamber at a first pressure, the filter cloth having a plurality of permeable perforations, thereby preliminarily removing a part of the liquid of the slurry through the perforation of the filter cloth and forming a filter cake;
inflating a resilient tube provided within the solid chamber to squeeze the filter cake at a second pressure and removing the liquid retained in the filter cake through the perforations of the filter cloth; and
discharging the filter cake from the chamber.

2. A method as defined in Claim 1, wherein the first pressure is at least 0.5 bar.

3. A method as defined in Claim 2, wherein the first pressure is from 1 bar to 12 bars.

4. A method as defined in Claim 1, wherein the second pressure is from 2 bars to 20 bars.

5. A method as defined in Claim 4, wherein the second pressure is from 4 bars to 12 bars.

6. A method as defined in any proceeding claim, wherein the perforations of the filter cloth are less than 3m³/m²min at the pressure of 200Pa.

7. A method as defined in any proceeding claim, wherein the resilient tube is inflated by introducing compressed air into the tube.

8. A method as defined in any of claims 1 to 6, wherein the resilient tube is inflated by introducing compressed water into the tube.

9. A method as defined in any proceeding claim, further comprising blowing compressed air through the filter cake before discharging to reduce moisture contents within the crystals.

10. A method as defined in Claim 9, wherein the compressed air is sterilized before blowing.

11. A method as defined in Claim 9, wherein the compressed air is heated before blowing.

12. A method according to any of claims 9 to 11, wherein the compressed air is blown at a pressure from 0.5 bars to 10 bars.

13. A method as defined in any preceeding claim, further comprising washing the filter cake with a liquid before discharging and subsequently inflating the tube at the second pressure to squeeze the filter cake and to remove the liquid.

14. A method as defined in Claim 13, wherein the liquid washing the filter cake before discharging is collected filtrate.

15. A method as defined in Claim 13, wherein the liquid washing the filter cake before discharging is sterilized water.

16. A method as defined in any preceeding claim, further comprising drying the filter cake after discharging to further remove moisture.

17. A method as defined in any preceeding claim, wherein the slurry is prepared by crystallizing the aspartame by evaporating solvent from an aspartame solution.

18. A method as defined in any of claims 1 to 16, wherein the slurry is prepared by crystallizing the aspartame by cooling an aspartame solution.

19. A method as defined in Claim 18, wherein the cooling crystallization is carried out with a forced flow of the solution.

20. A method as defined in Claim 18, wherein the cooling crystallization is carried out without a forced flow of the solution.

## Patentansprüche

1. Verfahren zur Isolierung von a-L-Aspartyl-L-phenylalaninmethylester- (Aspartam-) Kristallen aus einer Suspension, welche eine Flüssigkeit und die Aspartamkristalle darin suspendiert enthält, mit den Stufen, in denen man
die Aspartamsuspension herstellt;
die Aspartamsuspension in einen von einem Filtertuch eingeschlossenen Bereich innerhalb einer festen Kammer bei einem ersten Druck einbringt, wobei das Filtertuch eine Vielzahl von durchlässigen Perforationen aufweist und wobei man vorläufig einen Teil der Flüssigkeit der Suspension durch die Perforation des Filtertuchs entfernt und einen Filterkuchen ausbildet;
einen elastischen Schlauch, der innerhalb der festen Kammer vorgesehen ist, aufbläht, um den Filterkuchen bei einem zweiten Druck zusammenzudrücken und die in dem Filterkuchen zurückgebliebene Flüssigkeit durch die Perforationen des Filtertuches zu entfemen; und
den Filterkuchen aus der Kammer entnimmt.

2. Verfahren nach Anspruch 1, wobei der erste Druck wenigstens 0,5 bar beträgt.

3. Verfahren nach Anspruch 2, wobei der erste Druck von 1 bar bis 12 bar beträgt.

4. Verfahren nach Anspruch 1, wobei der zweite Druck von 2 bar bis 20 bar beträgt.

5. Verfahren nach Anspruch 4, wobei der zweite Druck von 4 bar bis 12 bar beträgt.

6. Verfahren nach einem der vorangegangenen Ansprüche, wobei die Perforationen des Filtertuches weniger als 3 m³/m² min bei dem Druck von 200 Pa betragen.

7. Verfahren nach einem der vorangegangenen Ansprüche, wobei der elastische Schlauch durch Einbringen von unter Druck stehender Luft in den Schlauch aufgeblasen wird.

8. Verfahren nach einem der Ansprüche 1 bis 6, wobei der elastische Schlauch durch Einbringen von unter Druck stehendem Wasser in den Schlauch aufgebläht wird.

9. Verfahren nach einem der vorangegangenen Ansprüche, welches weiterhin das Hindurchblasen von unter Druck stehender Luft durch den Filterkuchen vor der Entnahme zur Verminderung des Feuchtigkeitsgehalts innerhalb der Kristalle umfaßt.

10. Verfahren nach Anspruch 9, wobei die unter Druck stehende Luft vor dem Einblasen sterilisiert wird.

11. Verfahren nach Anspruch 9, wobei die unter Druck stehende Luft vor dem Einblasen erhitzt wird.

12. Verfahren nach einem der Ansprüche 9 bis 11, wobei die unter Druck stehende Luft bei einem Druck von 0,5 bar bis 10 bar eingeblasen wird.

13. Verfahren nach einem der vorangegangenen Ansprüche, welches weiterhin ein Waschen des Filterkuchens mit einer Flüssigkeit vor der Entnahme und anschließendes Aufblähen des Schlauches bei dem zweiten Druck zum Auspressen des Filterkuchens und zum Entfemen der Flüssigkeit umfaßt.

14. Verfahren nach Anspruch 13, wobei die Flüssigkeit, welche den Filterkuchen vor der Entnahme wäscht, gesammeltes Filtrat ist.

15. Verfahren nach Anspruch 13, wobei die Flüssigkeit, welche den Filterkuchen vor der Entnahme wäscht, sterilisiertes Wasser ist.

16. Verfahren nach einem der vorangegangenen Ansprüche, welches weiterhin ein Trocknen des Filterkuchens nach der Entnahme zur weiteren Entfernung von Feuchtigkeit umfaßt.

17. Verfahren nach einem der vorangegangenen Ansprüche, wobei die Suspension durch Kristallisieren des Aspartams durch Verdampfen von Lösungsmittel aus einer Aspartamlösung hergestellt wird.

18. Verfahren nach einem der Ansprüche 1 bis 16, wobei die Suspension durch Kristallisieren des Aspartams durch Abkühlen einer Aspartamlösung hergestellt wird.

19. Verfahren nach Anspruch 18, wobei die Kühlkristallisation unter einer erzwungenen Strömung der Lösung durchgeführt wird.

20. Verfahren nach Anspruch 18, wobei die Kühlkristallisation ohne eine erzwungene Strömung der Lösung durchgeführt wird.

## Revendications

1. Procédé pour isoler des cristaux d'ester méthylique d'α-L-aspartyl-L-phénylalanine (aspartame) d'une bouillie comprenant un liquide et des cristaux d'aspartame en suspension dans ce liquide, procédé comprenant les étapes consistant :
à préparer la bouillie d'aspartame
à introduire la bouillie d'aspartame dans une zone entourée par une toile de filtre à l'intérieur d'une chambre solide à une première pression, la toile de filtre comportant une pluralité de perforations perméables, en séparant ainsi préalablement une partie du liquide de la bouillie à travers la perforation de la toile de filtre et en formant un gâteau de filtre ;
à gonfler un tube résilient présent dans la chambre solide pour presser le gâteau de filtre à une seconde pression et à évacuer le liquide retenu dans le gâteau de filtre à travers les perforations de la toile de filtre ; et
à décharger le gâteau de filtre de la chambre.

2. Procédé répondant à la définition suivant la revendication 1, dans lequel la première pression est égale à au moins 0,5 bar.

3. Procédé répondant à la définition suivant la revendication 2, dans lequel la première pression est comprise dans l'intervalle d'1 bar à 12 bars.

4. Procédé répondant à la définition suivant la revendication 1, dans lequel la seconde pression est comprise dans l'intervalle de 2 bars à 20 bars.

5. Procédé répondant à la définition suivant la revendication 4, dans lequel la seconde pression est comprise dans l'intervalle de 4 bars à 12 bars.

6. Procédé répondant à la définition suivant l'une quelconque des revendications précédentes, dans lequel les perforations de la toile de filtre sont inférieures à 3 m³/m² min à la pression de 200 Pa.

7. Procédé répondant à la définition suivant l'une quelconque des revendications précédentes, dans lequel le tube résilient est gonflé en introduisant de l'air comprimé dans le tube.

8. Procédé répondant à la définition suivant l'une quelconque des revendications 1 à 6, dans lequel le tube résilient est gonflé en introduisant de l'eau comprimée dans le tube.

9. Procédé répondant à la définition suivant l'une quelconque des revendications précédentes, comprenant en outre le soufflage d'air comprimé à travers le gâteau de filtre avant de le décharger pour réduire la teneur en humidité à l'intérieur des cristaux.

10. Procédé répondant à la définition suivant la revendication 9, dans lequel l'air comprimé est stérilisé avant soufflage.

11. Procédé répondant à la définition suivant la revendication 9, dans lequel l'air comprimé est chauffé avant soufflage.

12. Procédé répondant à la définition suivant l'une quelconque des revendications 9 à 11, dans lequel l'air comprimé est soufflé à une pression comprise dans l'intervalle de 0,5 bar à 10 bars.

13. Procédé répondant à la définition suivant l'une quelconque des revendications précédentes, comprenant en outre le lavage du gâteau de filtre avec un liquide avant de le décharger et ensuite le gonflement du tube à la seconde pression pour presser le gâteau de filtre et pour évacuer le liquide.

14. Procédé répondant à la définition suivant la revendication 13, dans lequel le liquide lavant le gâteau de filtre avant de le décharger consiste en le filtrat recueilli.

15. Procédé répondant à la définition suivant la revendication 13, dans lequel le liquide lavant le gâteau de filtre avant de le décharger consiste en eau stérilisée.

16. Procédé répondant à la définition suivant l'une quelconque des revendications précédentes, comprenant en outre le séchage du gâteau de filtre après l'avoir déchargé pour une élimination supplémentaire d'humidité.

17. Procédé répondant à la définition suivant l'une quelconque des revendications précédentes, dans lequel la bouillie est préparée par cristallisation de l'aspartame en évaporant le solvant d'une solution d'aspartame.

18. Procédé répondant à la définition suivant l'une quelconque des revendications 1 à 16, dans lequel la bouillie est préparée par cristallisation de l'aspartame en refroidissant une solution d'aspartame.

19. Procédé répondant à la définition suivant la revendication 18, dans lequel la cristallisation par refroidissement est effectuée avec un courant forcé de la solution.

20. Procédé répondant à la définition suivant la revendication 18, dans lequel la cristallisation par refroidissement est effectuée sans courant forcé de la solution.
